# EUROPÄISCHE PATENTSCHRIFT

(11) **EP 1 083 884 B1**
(45) Veröffentlichungstag und Bekanntmachung des Hinweises auf die Patenterteilung: **16.10.2002**
(21) Anmeldenummer: 00917004.4
(22) Anmeldetag: 28.03.2000
(51) Int. Cl.: A61K 9/20, A61K 47/32, C08K 3/08

(54) **VERFAHREN ZUR STABILISIERUNG VON POLYVINYLPYRROLIDON-ZUBEREITUNGEN**
PROCESS FOR THE STABILISATION OF POLYVINYLPYRROLIDONE FORMULATIONS
PROCEDE DE PREPARATION DE POLYVINYLPYRROLIDONES STABILISEES

(30) Priorität: 06.04.1999 DE 19915420
(43) Veröffentlichungstag der Anmeldung: 21.03.2001
(73) Patentinhaber: BASF AKTIENGESELLSCHAFT, 67056 Ludwigshafen (DE)
(72) Erfinder: BÜHLER, Volker, D-67157 Wachenheim (DE); FILGES, Ulrich, D-67433 Neustadt (DE); SCHNEIDER, Tanja, D-64625 Bensheim (DE)
(86) Internationale Anmeldenummer: EP0002715
(87) Internationale Veröffentlichungsnummer: WO00059478

(56) Entgegenhaltungen:
- EP-A- 0 215 379
- EP-A- 0 832 846
- WO-A-91/12825
- GB-A- 1 513 258
- "Handbook of Pharmaceutical Excipients" 1986 , AMERICAN PHARMACEUTICAL ASSOCIATION & THE PHARMACEUTICAL SOCIETY OF GREAT BRITTAIN , ISBN: 0-917330-56-0 XP002145873 Seite 234 -Seite 239 & PATEL DM, ET AL.: "Povidone" Absätze [0010],[0012],[0017]

## Beschreibung

Die Erfindung betrifft ein Verfahren für die Herstellung von stabilisierten, als Hilfsmittel für die Arzneimittelformulierung geeigneten Polyvinylpyrrolidon- und/oder Polyvinylpyrrolidoncopolymere enthaltenden pulverförmigen Zubereitungen mit einem Zusatz von sehr geringen Mengen an Schwermetallen oder Peroxide spaltenden Enzymen, die nach der Polymerisation Zugegeben werden.

Polymere wie homopolymeres, lösliches oder unlösliches Polyvinylpyrrolidon ("Povidon" bzw. "Crospovidon") und das Vinylpyrrolidon-Vinylacetat-Copolymer ("Copovidon") werden nach ihrer Polymerisation durch Sprühtrocknung, Walzentrocknung oder einer anderen Warmlufttrocknung in rieselfähiges Pulver überführt. Bei diesen Prozessen bilden sich durch den intensiven Luftkontakt und die Wärme Spuren von Peroxiden, deren Gehalt im Laufe der darauffolgenden Verpackung und Lagerung noch weiter zunimmt. In den gültigen Pharmakopöen, z.B. in Ph. Eur. 3 und in JP XIII, ist der Peroxidgehalt für diese Substanzen auf maximal 400 ppm limitiert. Eine Trocknung unter Luftausschluß, eine Lagerung bei tiefen Temperaturen oder die hermetisch dichte Verpackung unter Vakuum oder einem Inertgas können zwar die Kinetik der Peroxidbildung verlangsamen, sie jedoch nicht verhindern.

Die genannten verschiedenen Typen von Polyvinylpyrrolidonpolymeren (Povidon, Crospovidon und Copovidon) werden weltweit als Hilfsstoffe in vielen verschiedenen Arzneiformen eingesetzt, wobei jedoch die Verwendung als Bindemittel und Sprengmittel in festen Formen, z.B. für Tabletten und Kapseln, im Vordergrund steht. In diesen Medikamenten liegen die Hilfsstoffe ebenfalls in fester Form vor und können während der Verarbeitung und der Lagerung zusammen mit empfindlichen Wirkstoffen deren Stabilität durch Oxidation negativ beeinflussen. Typische Beispiele sind Mutterkornalkaloide, Hormone, Antibiotika wie Doxycyclin, Metformin und Molsidomin. Der Verwendung von Polyvinylpyrrolidon in Kombination mit derartigen oxidationsempfindlichen Wirkstoffen sind aus diesem Grund enge Grenzen gesetzt. Deshalb kann in diesen Fällen meist nur ein frisch hergestelltes Polyvinylpyrrolidon verwendet werden, dessen Peroxidgehalt durch die Lagerung noch nicht weiter angestiegen ist. Die Bereitstellung eines solchen Materials bereitet in vielen Ländern der Welt jedoch oft große logistische Probleme.

Es bestand daher die Aufgabe, ein Verfahren zur Stabilisierung Polyvinylpyrrolidon enthaltender Hilfsmittel vorzuschlagen, die einen stark verringerten Peroxidgehalt aufweisen.

Diese Aufgabe wurde erfindungsgemäß gelöst mit einem Verfahren zur Stabilisierung von als Hilfsmittel zur Arzneimittelformulierung geeigneten, lösliches oder unlösliches Polyvinylpyrrolidon oder Polyvinylpyrrolidoncopolymere enthaltenden oder daraus bestehenden pulverförmigen Zubereitungen gemäß Anspruch 1, dadurch gekennzeichnet, daß man den wäßrigen Lösungen der Polymere sehr geringe Mengen Schwermetalle, die in den angewendeten Mengen physiologisch verträglich sind, oder Peroxide spaltende Enzyme in sehr geringen Mengen nach der Polymerisation zusetzt und das Polymer durch Trocknung in die Pulverform überführt.

In der Fachliteratur ist zwar bekannt, daß Polyvinylpyrrolidon in Analogie zu Iod auch mit Metallen stöchiometrische Komplexe bildet (z.B. mit Kobalt, Nickel und Mangan) und daß diese Komplexe in wäßriger Lösung Wasserstoffperoxid katalytisch bei einem pH-Wert von über 7,3 zersetzen (S.Sh. Rashidova, M.D. Inoyatov, Usb. Khim. Zh 3, Seiten 32-44, 1977)). Genauso ist bekannt, daß Wasserstoffperoxid in Gegenwart von Polyvinylpyrrolidon spezifisch durch Peroxidase oder Katalase in Lösung abgebaut wird (WO 9415648). Verfahren für die Zubereitung von Polyvinylpyrrolidon sind bekannt, zum Beispiel von GB1 513 258. In allen in der Literatur beschriebenen Fällen werden jedoch hohe Konzentration der Metallkomplexe und der Enzyme in Lösung angewendet.

Es war nun überraschend, daß äußerst geringe Mengen, in der Regel weniger als 10 ppm der Schwermetalle, nicht nur in der Lösung die Peroxidkonzentration herabsetzen, sondern auch noch in der Pulverform stabilisierend wirken, so daß bei Lagerung der Peroxidgehalt praktisch nicht mehr ansteigt, wie dies bei der nicht stabilisierten Zubereitung der Fall ist. Dies ist insofern erstaunlich, als in der festen Zustandsform nur eine topochemische Reaktion möglich ist und bei der großen Verdünnung des Stabilisators nicht zu erwarten war, daß eine Reaktion mit den neu gebildeten ebenfalls in geringer Konzentration vorliegenden Peroxiden überhaupt möglich ist. Schließlich findet sich in der Literatur die Information, daß Polyvinylpyrrolidon nicht nur Enzyme stabilisieren, sondern auch inhibieren kann, so daß auch deshalb die regelmäßige vorteilhafte Wirkung der erfindungsgemäß zugesetzten Enzyme überraschend war.

Für den Zusatz der Schwermetalle und/oder Enzyme ergibt sich eine relativ enge Bandbreite. Einerseits ist eine bestimmte Wirkungsmenge erforderlich. Andererseits sind aus Gründen der physiologischen Verträglichkeit nach oben Grenzen gesetzt.

In der Regel wird man daher einen Zusatz von 10 ppm Schwermetalle (bezogen auf Gewichtsmenge Metalle pro Gewichtsmenge Hilfsmittelpulver) nicht überschreiten. Bevorzugt sind Mengen von 1 bis 10 ppm und insbesondere 5 bis 8 ppm.

Bei den Enzymen ist aus Gründen der physiologischen Verträglichkeit die Obergrenze nicht kritisch. Bevorzugt sind Mengen von 0,1 bis 20, vorzugsweise 0,5 bis 10 ppm.

Als Schwermetalle kommen z.B. Mangan, Zink, Kobalt und insbesondere Kupfer in Betracht.
Peroxidespaltende Enzyme, die erfindungsgemäß verwendet werden, sind insbesondere Katalase und Peroxidase.

Zur Herstellung der stabilisierten Zubereitungen geht man zweckmäßig so vor, daß man die Schwermetalle in Form von deren in Wasser löslichen Salze, z.B. der Sulfate oder Chloride der Lösung oder Suspension der Polymeren zusetzt und die Lösung bzw. Suspension trocknet. Obgleich die Trocknung auf beliebige Art vorgenommen werden kann, sind Sprühtrocknung oder Walzentrocknung bevorzugt.

Im übrigen sind diese Methoden der Herstellung der Vinylpyrrolidon enthaltenden Hilfsmittel bekannt und es wird auf Einzelheiten in der Standard-Literatur verwiesen.

Auch die chemischen und physikalischen Eigenschaften der als Hilfsmittel zu verwendenden löslichen (a) Polyvinylpyrrolidone (Povidon), unlöslichen (b) Polyvinylpyrrolidone (Crospovidon) und (c) Polyvinylpyrrolidoncopolymere, das sind z.B. Vinylpyrrolidon-Vinylacetat-Copolymere (Copovidon), sind in der Literatur im einzelnen beschrieben.

Unter (a) werden im allgemeinen Polymere mit einem mittleren Molekulargewicht M_{W} von 2000 bis 1500000 (= K-Wert 10 bis 96) verstanden, wie sie im Kommentar des Europäischen Arzneibuchs, 9. Lieferung 1998, Monographie "Povidonum", im einzelnen beschrieben sind. Auf diese Definition wird ausdrücklich Bezug genommen, und die dort gemachten Angaben sollen als hier inkorporiert gelten.

Unter (b) werden Popcorn-Polymere verstanden, deren mittleres Molekulargewicht aufgrund ihrer vollständigen Unlöslichkeit in allen Lösemitteln nicht bestimmbar ist. Da das IR-Spektrum mit dem von Povidon (a) praktisch identisch ist, kann man davon ausgehen, daß es sich um weitgehend physikalisch vernetzte Homopolymere des Vinylpyrrolidons handelt, wie sie in dem Buch "Kollidon, Polyvinylpyrrolidone for the pharmaceutical industry" 4. Ausgabe 1999, BASF Aktiengesellschaft, beschrieben sind.

Unter (c) versteht man lösliche oder teilweise unlösliche Copolymere von Vinylimidazol, Vinylcaprolactam mit Vinylpyrrolidon, C₁₋₁₈-Alkylester von Acrylsäure und Methacrylsäure mit Vinylpyrrolidon, C₁₋₁₈-Alkylvinylester von (Meth)acrylsäure mit Vinylpyrrolidon. Unter C₁₋₁₈-Alkylestern der Acrylsäure bzw. Methacrylsäure versteht man z.B. Ethylacrylat, tert.-Butylacrylat.

C₁₋₁₈-Alkylvinylester können z.B. sei Vinylacetat, Vinylpropionat, Stearyl(meth)acrylat, Crotyl(meth)acrylat. Bevorzugt sind Copolymere von Vinylacetat und Vinylpyrrolidon mit einem K-Wert von 20 bis 50, deren Löslichkeit vom Verhältnis der beiden Monomeren abhängt. Bevorzugt wird in diesem Fall das Verhältnis von 6 Teilen Vinylpyrrolidon und 4 Teilen Vinylacetat, das ein noch wasserlösliches Polymer mit einem K-Wert 25 bis 35 ergibt, wie es ebenfalls in dem Buch "Kollidon, Polyvinylpyrrolidone for the pharmaceutical industry" 4. Ausgabe 1999, BASF Aktiengesellschaft, beschrieben ist.

Das erfindungsgemäße Verfahren zur Stabilisierung kommt besonders für solche Zubereitungen in Betracht, die für die Herstellung von Tabletten Verwendung finden sollen. Im Vordergrund steht dabei der Einsatz von Povidon (a) und Copovidon (c) als Tablettenbindemittel mit Feuchtgranulaten oder als Trockenbindemittel. Im einzelnen wird auf das Buch verwiesen "Kollidon, Polyvinylpyrrolidone for the pharmaceutical industry" 4. Ausgabe 1999, BASF Aktiengesellschaft.

Eine weitere Anwendung besteht in der Bereitstellung von stabilisierten Hilfsmitteln als Sprengmittel von Tabletten. Dafür kommt vor allem Crospovidon (b) in Betracht.

Entsprechende Tablettenformulierungen bestehen z.B. aus Wirkstoff, Füllstoff, Povidon oder Copovidon als Bindemittel, Crospovidon als Sedimentationsinhibitor, Saccharose als Füll- und Geschmacksmittel, Zitronensäure als ionogene Komponente, Konservierungsmittel und Wasser.

### Beispiel 1

Eine Lösung von Povidon K25 (Polyvinylpyrrolidon; K-Wert 25) wurde mit verschiedenen Mengen einer 0,01 %igen Kupferchloridlösung versetzt und bei 100°C getrocknet. Das erhaltene Pulver wurde gemahlen und bei Raumtemperatur unter Luftatmosphäre verschlossen gelagert.

**Tabelle 1**

| Zugesetzte Kupfermenge (bezogen auf das Povidon-Pulver) | Peroxidgehalt nach Lagerung [ppm] | | | |
|---|---|---|---|---|
| | Nach Trocknung | Nach 6 Monaten | Nach 12 Monaten | Nach 24 Monaten |
| 2 ppm | 58 | 253 | 276 | 322 |
| 4 ppm | 69 | 184 | 184 | 253 |
| 6 ppm | 69 | 69 | 69 | <50 |
| 8 ppm | 69 | 58 | <50 | <50 |

Aus Tabelle 1 geht hervor, daß bei Kupferzugaben bis zu 4 ppm nur eine geringe Verhinderung der Peroxidbildung erfolgt. Bei Zusatz von 6 ppm Kupfer bleibt der Peroxidgehalt während der Lagerung konstant und bei 8 ppm nimmt er sogar im Laufe der Lagerzeit ab.

### Beispiel 2

Eine Lösung von Povidon K25 wurde mit verschiedenen Mengen von 0,01 %igen Lösungen von Kupferchlorid und Cobaltchlorid (1:1) versetzt und bei 100°C getrocknet. Das erhaltene Pulver wurde gemahlen und bei Raumtemperatur unter Luftatmosphäre verschlossen gelagert.

**Tabelle 2**

| Zugesetzte Metallmenge (bezogen auf das Povidon-Pulver) | Peroxidgehalt nach Lagerung [ppm] | | | |
|---|---|---|---|---|
| | Nach Trocknung | Nach 6 Monaten | Nach 12 Monaten | Nach 24 Monaten |
| 1 ppm Cu + 1 ppm Co | 103 | 207 | 230 | 253 |
| 3 ppm Cu + 3 ppm Co | 69 | 184 | 270 | 276 |
| 4 ppm Cu + 4 ppm Co | 69 | 126 | 207 | 207 |
| 5 ppm Cu + 5 ppm Co | 69 | 69 | 58 | 69 |

Tabelle 2 zeigt, daß Kupfer allein eine höhere Wirkung zeigt.

### Beispiel 3

Eine Lösung von Povidon K30 wurde mit verschiedenen Mengen einer 0,01 %igen Kupferchloridlösung versetzt, sprühgetrocknet und das Pulver offen bei Raumtemperatur gelagert.

**Tabelle 3**

| Zugesetzte Kupfermenge (bezogen auf das Povidon-Pulver) | Peroxidgehalt nach Lagerung [ppm] | | | |
|---|---|---|---|---|
| | Nach Trocknung | Nach 1 Monat | Nach 2 Monaten | Nach 6 Monaten |
| 0 ppm | 69 | 138 | 161 | 230 |
| 4,7 ppm | 115 | 115 | 115 | 92 |
| 9,4 ppm | 69 | 46 | 46 | 23 |

### Beispiel 4

Eine Lösung eines Copolymeren aus 70 Gew.-% Vinylpyrrolidon und 30 Gew.-% Stearylmethacrylat wurde mit verschiedenen Mengen einer 0,01 %igen Kupferchloridlösung versetzt und bei 100°C getrocknet. Das erhaltene Produkt wurde bei Raumtemperatur unter Luftatmosphäre verschlossen gelagert.

**Tabelle 4**

| Zugesetzte Kupfermengen (bezogen auf Pulver) [ppm] | Peroxidgehalt nach Trocknung [ppm] | Peroxidgehalt nach 4 Wochen Lagerung [ppm] |
|---|---|---|
| 0 | 30 | 62 |
| 7 | 30 | 24 |
| 10 | 30 | 22 |

Aus Tabelle 4 geht hervor, daß die Verhinderung der Peroxidbildung mittels Kupferzusatz nicht auf Homopolymere des Vinylpyrrolidons beschränkt ist, sondern auch bei dessen Copolymeren eine vergleichbare Wirkung zeigt.

### Beispiel 5

Im folgenden Beispiel wird aufgezeigt, daß bereits kleinste Zusätze des Enzyms Katalase mit den in Polyvinylpyrrolidon gebildeten Peroxiden reagiert und diese abbaut.

Hierzu wurden jeweils 50 g einer etwa 30 %igen wäßrigen Lösung von zwei verschiedenen Chargen von Povidon K25 mit unterschiedlichem Peroxidgehalt (pH-Wert 3,0 bis 5,0) mit verschiedenen Mengen von 0,1 und 0,001 %igen wäßrigen Lösungen von Katalase versetzt, 1 Stunde bei Raumtemperatur stehengelassen und deren Peroxidgehalt gemäß der Methode der Ph.Eur.-Monographie "Povidon" gemessen.

**Tabelle 5**

| Zugesetzte Katalase-Menge, berechnet als Pulver | Peroxidgehalt, bezogen auf die Lösung [ppm] | |
|---|---|---|
| | 1. Povidon-Charge | 2. Povidon-Charge |
| ohne Zusatz | 124 | 59 |
| 1 µg (0,07 ppm) | 122 | 57 |
| 10 µg (0,7 ppm) | 89 | 42 |
| 100 µg (6,7 ppm) | 26 | 26 |

## Patentansprüche

1. Verfahren zur Stabilisierung von als Hilfsmittel zur Arzneimittelformulierung geeigneten, lösliches oder unlösliches Polyvinylpyrrolidon oder Polyvinylpyrrolidoncopolymere enthaltenden oder daraus bestehenden pulverförmige Zubereitungen , **dadurch gekennzeichnet, daß** man den wäßrigen Lösungen der Polymere sehr geringe Mengen Schwermetalle, die in den angewendeten Mengen physiologisch verträglich sind, oder Peroxide spaltende Enzyme in sehr geringen Mengen nach der Polymerisation zusetzt und das Polymer durch Trocknung in die Pulverform überführt.

2. Verfahren gemäß Anspruch 1, **dadurch gekennzeichnet, daß** man bis zu 10 ppm der Schwermetalle oder bis zu 20 ppm Enzyme zusetzt, bezogen auf Gewichtmenge Metalle pro Gewichtmenge Hilfsmittelpulver.

3. Verfahren gemäß Anspruch 1, **dadurch gekennzeichnet, daß** man der Lösung des Polymers ein lösliches Kupfersalz, Peroxidase oder Katalase zusetzt.

4. Verfahren gemäß Anspruch 1, **dadurch gekennzeichnet, daß** man die Trocknung durch Sprühtrocknung oder Walzentrocknung vornimmt.

5. Verfahren gemäß Anspruch 1, **dadurch gekennzeichnet, daß** man der Lösung des Polymers 0.1 bis 20 ppm Katalase oder Peroxydase zusetzt, bezogen auf Gewichtmenge Enzyme pro Gewichtmenge Hilfsmittelpulver.

## Claims

1. A method for stabilizing a preparation in powder form comprising or consisting of soluble or insoluble polyvinylpyrrolidone or polyvinylpyrrolidone copolymers and suitable as aid for pharmaceutical formulations, which comprises adding to the aqueous solutions of the polymers very small amounts of heavy metals which are physiologically tolerated in the amounts used, or peroxide-cleaving enzymes in very small amounts after polymerization, and converting the polymer into the powder form by drying.

2. A method as claimed in claim 1, wherein up to 10 ppm of heavy metals or up to 20 ppm enzymes are added, based on amount of metal by weight per amount of powdered aid by weight.

3. A method as claimed in claim 1, wherein a soluble copper salt, peroxidase or catalase is added to the solution of the polymer.

4. A method as claimed in claim 1, wherein the drying is carried out by spray drying or drum drying.

5. A method as claimed in claim 1, wherein from 0.1 to 20 ppm catalase or peroxidase, based on amount of metal by weight per amount of powdered aid by weight, are added to the solution of the polymer.

## Revendications

1. Procédé pour stabiliser des compositions pulvérulentes consistant en totalité ou en partie en polyvinylpyrrolidone ou copolymères de la vinylpyrrolidone solubles ou insolubles, et convenant en tant que produit auxiliaire pour des compositions de médicaments, **caractérisé par le fait que**, après la polymérisation, on ajoute aux solutions aqueuses des polymères de très petites quantités de métaux lourds, non toxiques aux quantités mises en oeuvre, ou des enzymes dégradant les peroxydes en très faibles quantité, et on met le polymère à l'état pulvérulent par séchage.

2. Procédé selon la revendication 1, **caractérisé par le fait que** l'on ajoute jusqu'à 10 ppm de métaux lourds ou jusqu'à 20 ppm d'enzymes, les proportions indiquées pour les métaux s'entendant en poids et par rapport au poids de la poudre de produit auxiliaire.

3. Procédé selon la revendication 1, **caractérisé par le fait que** l'on ajoute à la solution du polymère un sel de cuivre soluble, une peroxydase ou une catalase.

4. Procédé selon la revendication 1, **caractérisé par le fait que** l'on sèche par atomisation ou sur cylindre.

5. Procédé selon la revendication 1, **caractérisé par le fait que** l'on ajoute à la solution du polymère 0,1 à 20 ppm de catalase ou de peroxydase, ces proportions s'entendant en poids pour les enzymes et par rapport au poids de la poudre de produit auxiliaire.
